# EUROPEAN PATENT SPECIFICATION

(11) **EP 2 618 834 B1**
(45) Date of publication and mention of the grant of the patent: **06.01.2016**
(21) Application number: 11761058.4
(22) Date of filing: 21.09.2011
(51) Int. Cl.: A61K 39/00, A61K 39/39

(54) **A METHOD FOR KEEPING A FOOT-AND-MOUTH DISEASE VACCINE AVAILABLE FOR EMERGENCY VACCINATION**
VERFAHREN ZUR BEREITHALTUNG EINES IMPFSTOFFES GEGEN MAUL- UND KLAUENSEUCHE FÜR NOTFALLIMPFUNGEN
PROCÉDÉ POUR GARDER UN VACCIN CONTRE LA FIÈVRE APHTEUSE DISPONIBLE POUR UNE VACCINATION D'URGENCE

(30) Priority: 24.09.2010 US 890103; 22.09.2010 EP 10178454
(43) Date of publication of application: 31.07.2013
(73) Proprietor: Intervet International B.V., 5831 AN Boxmeer (NL)
(72) Inventor: GOOVAERTS, Daniel G.E., 5831 AN Boxmeer (NL); JANSEN, Theodorus, 5831 AN Boxmeer (NL); PAUL, Guntram, 50739 Köln (DE)
(74) Representative: Intervet International B.V.
(86) International application number: PCT/EP2011/066373
(87) International publication number: WO 2012/038452

(56) References cited:
- EP-A1- 1 201 251
- WO-A1-2010/133592
- WO-A2-01/45670
- BARNETT P V ET AL: "Stratified and cryogenically stored (SACS) vaccines, a new concept in emergency foot-and-mouth disease vaccine formulation and storage", VACCINE, BUTTERWORTH SCIENTIFIC. GUILDFORD, GB, vol. 20, no. 16, 15 May 2002 (2002-05-15), pages 2060-2064, XP004349564, ISSN: 0264-410X
- COX SARAH J ET AL: "Experimental evaluation of foot-and-mouth disease vaccines for emergency use in ruminants and pigs: a review", VETERINARY RESEARCH (LES ULIS), vol. 40, no. 3, May 2009 (2009-05), XP007916907, ISSN: 0928-4249
- P. SUTMÖLLER, IVO GOMES, D. ABARACÓN: "Storage of inactivated oil adjuvanted Foot-and-Mouth disease vaccine at low temperature", BOL. CENTRO PANAMERICANO FIEBRE AFTOSA, vol. 51, 1 January 1985 (1985-01-01), pages 37-38, XP55115087,
- D. ABARACON, J.A. MESQUITA, S. SALLUA, R. PEREZ RAMA: "Montanide 888 emulsifying agent for preparation of oil adjuvanted foot-and-mouth disease vaccine", BOL. CENTRO PANAMERICANO FIEBRE AFTOSA, vol. 45-46, 1 January 1982 (1982-01-01), pages 55-56, XP55115219,
- TERRETT ET AL: "Combinatorial Chemistry Online", COMBINATORIAL CHEMISTRY - AN ONLINE JOURNAL, ELSEVIER, vol. 8, no. 6, 1 June 2006 (2006-06-01), pages 27-30, XP005511508, ISSN: 1464-3383, DOI: 10.1016/J.COMCHE.2006.05.001

## Description

The present invention pertains to a method for keeping a foot-and-mouth disease vaccine available for emergency vaccination of animals against foot-and-mouth disease, the vaccine comprising a foot-and-mouth disease antigen formulated in a water-in-oil emulsion. The invention is broadly defined in the appended claims.

A foot-and-mouth disease (FMD) vaccine comprising a foot-and-mouth disease antigen formulated in a water-in-oil emulsion is known from the prior art. For example AFTOVACIN OLEOSA™, available from Intervet Schering-Plough animal health Brazil, is such a vaccine. Typically, formulated FMD vaccines are stored at a temperature of about 4°C to be available immediately in case of emergency vaccination programs. A very important disadvantage of formulated FMD vaccines in general is that the antigens are relatively unstable per se. The potency of formulated vaccines therefore, even when stored at 4°C, decreases significantly over time. In many cases, even in a period as short as 6 to 18 months, the potency of the vaccine may fall below the registered level and if so, the vaccine should be replaced by a fresh batch. This is not only inconvenient, but also economically unattractive. The development of ultra cold storage of concentrated antigens, which can be specifically formulated into a vaccine, as and when required, has become the more accepted strategy. This strategy has led to the establishment of national and shared antigen banks (see Vet. Res. 2009, 40:13; Experimental evaluation of foot-and-mouth disease vaccines for emergency use in ruminants and pigs: a review).

The strategy of ultra cold storage however also has some disadvantages. The most pronounced one is that it takes considerable time to formulate the vaccine. Typically it takes 72 to 120 hours to formulate the vaccine and ship it to end-users. In case of an emergency, this is considered a long time. Another disadvantage is that the potency of the composition cannot be tested in these emergency circumstances, such tests would simply take too much time. Therefore, vaccines prepared from ultra cold stored antigens are usually formulated to contain higher levels of antigen than conventional prophylactic vaccines, and as a consequence are expected to provide the required immune response. Still, the actual potency can never be established before the vaccine is used. Moreover, the safety also remains uncertain. Indeed, the lack of precision and inherent variability is a concern with these vaccines.

Freezing FMD vaccines, although in theory usable to overcome the disadvantages of the current emergency vaccines made of ultra cold stored antigens (namely: freezing and subsequent thawing allows almost immediate availability of the vaccine, and potency and safety can be tested beforehand), is not regarded as a viable option to keep a foot-and-mouth disease vaccine available for emergency vaccination. Although in the past it has been mentioned (*Bol. Centro Panamericano Fiebre Aftosa 51*: 35-36, 1985) that one specific oily FMD vaccine (the type of emulsion however is not mentioned, nor the method to obtain the emulsion) could be stored at -70°C without loss of potency, in the light of present knowledge those results are regarded as erroneous. In particular, Barnett in Vaccine 20 (2002), pp 2060-2064, states that work at the Institute for Animal health, Pirbright, has shown that a reduction in potency occurs when oil adjuvanted vaccines are stored at either -20°C or -70°C, and therefore "neither type of formulation [oil or aqueous] can be frozen under these conditions without detrimental effect on their potency". Indeed, in the article in Vaccine it is shown that the potency of an oil-in-water vaccine based on the oil ISA 25 (available from Seppic, France), decreases tremendously when the formulation is snap-frozen and thawed immediately afterwards (see Table 4, page 2063). In the 1985 disclosure mentioned here-above the potency appears to be increased after more than four years of frozen storage. This is in complete contradiction with the **Vaccine** article, and moreover, is technically even impossible given the inherent instability of FMD antigens even when stored at temperatures as low as -70°C. The skilled person in 2010 will thus be convinced that the results in the 1985 disclosure are erroneous. Next to this, since the 1985 disclosure gives no clear method to produce the oily adjuvant, the tests described cannot be repeated. In short, the 1985 disclosure is no incentive at the priority date of the present application to search for an FMD vaccine, based on a W/O emulsion that can be stored frozen. Indeed, an alternative freezing strategy has been developed in the art, which method is described in the **Vaccine** article mentioned here-above and in EP 1 201 251. In this method, the FMD vaccine is kept available for emergency vaccination by providing a stratified cryogenically stored vaccine (also called "SACS"). This method is based on an approach of layering the individual components of a vaccine in the same primary container and then storing the product at ultra-low temperatures (around - 150°C). Although with this method a detrimental effect on potency is avoided, the method is very complex and the fact that the vaccine has to be stored at ultra-low temperatures makes the method very expensive to apply. It is also noted that in the Vaccine article an emulsion based on Montanide ISA 206 can be snap-frozen without loss of potency. This emulsion however is an oil-in-water emulsion (O/W) which only after administration to a subject animal transfers into a water-in-oil-in-water emulsion (W/O/W). The freezing of such a O/W emulsion without loss of potency is also described in the international patent application WO 2010/133592 A1. It is commonly known that the effects of freezing on the emulsion quality depends on the type of emulsion. The present invention pertains to water-in-oil emulsions.

It is noted that the freezing of a formulated vaccine based on a water-in-oil emulsion is mentioned in WO 01/45670. However, the type of antigen used in this patent application is a peptide attached to an immunogenic protein carrier such as a diphtheria or tetanus toxoid. Such preparations do hardly depend on the presence or quality of an adjuvant emulsion since they are highly immunogenic per se. Indeed, a vaccine comprising an oily adjuvant based on ISA 25 is mentioned as a suitable for freezing (se page 7, line 24). For FMD antigens however, the art has proven specifically that this is not the case for a vaccine based on the oily adjuvant ISA 25 (see Vaccine article, mentioned here-above). In other words, the art in the area of FMD recognizes that the alleged general teaching as disclosed in WO 01/45670 is not applicable to FMD vaccines, in particular not to FMD vaccines based on oily adjuvant emulsions.

### OBJECT OF THE INVENTION

It is an object of the invention to overcome or at least mitigate prior art problems pertaining to the availability of FMD vaccines for emergency vaccination, and to provide a convenient way of keeping a formulated FMD vaccine available for use. This is done by providing a method as defined in the appended claims.

### DEFINITIONS

Snap-freezing: a process by which the temperature of a sample is very quickly lowered to temperatures below -140°C by submerging the sample in a liquid having a temperature below -140°C (in particular liquid nitrogen at -196°C) or the gaseous phase of such a liquid.

Freezing around -20°C: to freeze under conditions similar to freezing in a standard house-hold compressor type freezer, having an inner temperature (in the freezing compartment) typically between -12 and -28°C.

Potency: a measure of the capacity to trigger the immune system of a host.

Vaccine: a constitution suitable for application to an animal (including humans), comprising one or more antigens (such as attenuated or killed microorganisms and/or subunits thereof, or any other substance such as a metabolite of an organism), in an immunologically effective amount (i.e. capable of stimulating the immune system of the target animal), typically combined with a pharmaceutically acceptable carrier (such as for example a liquid containing water), optionally comprising immunostimulating agents (adjuvants), which upon administration to the animal induces an immune response which suffices to treat a disease or disorder of the animal, i.e. to at least aid in preventing, ameliorating or curing the disease or disorder.

Adjuvant: a substance or composition that is able to favor or amplify a particular process in the cascade of immunological events, ultimately leading to a better immunological response against an antigen.

To ship: to cause to be conveyed to a destination, for example using ordinary mail or express carriage, using road haulage, air transport, transport over water or whatever means suitable for a package or other tangible item.

### DESCRIPTION OF THE INVENTION

Surprisingly applicant has found that, against the common knowledge in the art of FMD vaccines one can freeze an FMD vaccine, when based on a water-in-oil emulsion, without detrimental effect on the potency of the vaccine. The advantage of the presently found effect is that the vaccine can be stored at significantly lower temperatures than 4°C, and thus that the natural deterioration speed of the FMD antigens in the composition can inherently be slowed down (or even virtually blocked) as is commonly known from the cold storage of the antigens per se. Indeed, in a co-pending application (PCT/EP2010/056815; Tables 1, 2 and 3 and accompanying description) it has been experimentally established that the potency of a formulated vaccine, once frozen, hardly deteriorates in a period of 12 months. Applicant thus found a very convenient and simple way to keep a formulated FMD vaccine available for emergency vaccination. The very important advantages are self-evident: one can now store a tested (potency and safety) FMD vaccine, and have the vaccine, without a detrimental loss of potency, available in an emergency situation within hours by simply shipping it to the place where it should be administered, and thawing it before administration.

In an embodiment a potency of the vaccine is established before it is stored. It is very advantageous when the potency of the vaccine is known before administration. In the prior art, when FMD vaccine is formulated based on ultra cold stored antigens in case of an emergency situation, there is not time left to assess the potency of the vaccine. With the present invention, use can be made of a validated vaccine, also in case of emergency vaccination.

In an embodiment the vaccine is supplied to a third party (also covering a mailing address that is different from the place of residence of the third party), typically upon its request, before it is frozen. In this embodiment, the manufacturer does not need to freeze the vaccine before it is shipped to a third party. The said third party may store the vaccine itself in frozen condition. This embodiment is for example advantageous when the third party envisages that large amounts of the vaccine may need to be available immediately when an emergency situation arises and no time should be lost. In that case the party may thus desire to keep a validated FMD vaccine available for vaccination under its own control, for a long period of time.

Alternatively, the vaccine is supplied to a third party after it has been frozen. In this embodiment it may be that the manufacturer of the vaccine is the party that stores the vaccine in frozen condition. This has the advantage that the manufacturer may have permanent control over the circumstances under which the vaccine is stored until it is shipped to a third party.

In all embodiments the vaccine is frozen by using a non snap-freezing process. A snap-freezing process has the important disadvantage that specific, often expensive apparatus is needed. Such apparatus may not be available at each and every location at which a party wants to store the vaccine. Moreover, a snap- freezing process requires trained operators in view of the risks involved when working with liquids at ultra-low temperatures (choking, burning wounds etc). Applicant surprisingly found that in the present invention, one can make use of a non snap-freezing process to freeze the vaccine. It has been found even that a conventional compressor type freezer, such as an ordinary house-hold type freezer, will suffice. Although the freezing time is significantly longer in such a freezer, and thus the negative impact on potency may be expected to be tremendous, applicant surprisingly found that in the present method this has no or hardly any negative influence on the potency of the FMD vaccine. It has even appeared to be adequate when freezing takes place around -20°C, i.e. at a freezing temperature as present in an ordinary house-hold type freezer.

In an embodiment the water-in-oil emulsion is based on mineral oil. It has been found that the use of mineral oil, in particular an oil comprising saturated hydrocarbons such as Marcol 52™, can be very advantageously used in the present invention. In particular, neutralizing antibody titers can be obtained which are higher than obtainable with a commercially available FMD vaccine based on an oil-in-water emulsion adjuvant.

As stated here-above, the present invention also pertains to a method for testing a foot-and-mouth disease vaccine comprising a foot-and-mouth disease antigen formulated in a water-in-oil emulsion, the method comprising formulating the vaccine, freezing the vaccine, storing the frozen vaccine for a predetermined time, thawing the vaccine, and determining the potency of the thawed vaccine.

The invention also pertains to a combination of a foot-and-mouth disease vaccine comprising a foot-and-mouth disease antigen formulated in a water-in-oil emulsion, and an instruction that the vaccine may be stored at a temperature below a freezing temperature of the vaccine, until needed for vaccination. In an embodiment the combination is a package (for example a box, vial, flask or any other container) comprising the vaccine and the instruction in tangible form (for example in the form of a leaflet, printed instructions on the package or in the form of a sticker glued to the package). In an alternative embodiment the instruction is a recommendation made public via oral or written description, for example the recommendation that the FMD vaccine may be frozen and stored in that condition until needed for administration. The recommendation might for example be shown on a web-site of a distributor or manufacturer of an FMD vaccine, or is made public via an article in a journal, presentation on a congress or other equivalent happing, or is presented, either solely via oral means or in written form or a combination of oral and written form, in a commercial presentation such as a television-commercial or a presentation in a booth of a medical or scientific gathering.

### EXAMPLES

The effect of freezing on the potency of several inactivated FMD vaccines formulated with different water-in-oil adjuvants was tested. In order to allow adequate assessment, all adjuvants are based on the same oil, viz. medicinal grade white oil Marcol 52 (available from ExxonMobil Chemical ltd, Southampton, UK). The oil as such will not interfere in the freezing process and thus the results are applicable to other oils as well. A parameter that is believed to possibly interfere with the freezing process is the viscosity of the emulsion. Therefore, three adjuvant formulations were made covering a large part of the spectrum of viscosities for injectable emulsions (30-450 mPa.s).

As a benchmark, a vaccine was formulated that comprises the same adjuvant emulsion as the commercially available FMD vaccine DECIVAC® FMD DOE (available from Intervet/Schering-Plough Animal Health, Boxmeer, The Netherlands). This newly formulated benchmark vaccine comprised inactivated FMD antigens of O1 Manisa (5µg/ml), A Turkey (4 µg/ml) and Asia 1 Shamir (4 µg/ml). This benchmark vaccine was stored at 4°C.

To study the effect of freezing on a W/O (water-in-oil) emulsions, three experimental adjuvant emulsions were made, depicted in Table 1.

**Table 1 adjuvant emulsions**

| | emulsion 1 | emulsion 2 | emulsion 3 |
|---|---|---|---|
| water/oil (v/v) | 30/70 | 45/55 | 50/50 |
| stabilizers | sorbitan monooleate esters | sorbitan monooleate esters | Montanide 888 (Seppic, France) |
| viscosity (mPa.s) | 40 | 120 | 300 |

With these adjuvant emulsions, the following vaccines were made.

A first vaccine (designated vaccine A), based on emulsion 1, and containing FMD antigens of O1 Manisa (5µg/ml), A Turkey (4 µg/ml) and Asia 1 Shamir (4 µg/ml).

A second vaccine (designated vaccine B), based on emulsion 2, and containing FMD antigens of O1 Manisa (5µg/ml), A Turkey (4 µg/ml) and Asia 1 Shamir (4 µg/ml).

A third vaccine (designated vaccine C), based on emulsion 3, and containing FMD antigens of O1 Manisa (5µg/ml) and A Turkey (4 µg/ml).

To assess the effect of freezing, batches of freshly prepared vaccines A, B and C were either stored at 4°C, or stored in a standard compressor type household freezer at - 18°C for approximately 2 months. The vaccines were warmed to about 37°C immediately before administration.

Seven groups of six guinea pigs were used to perform the experiment. The pigs were placed in an isolated room and were vaccinated intramuscularly with 0.2 ml vaccine. Four weeks after the vaccination, blood samples were taken individually from all Guinea pigs. Serum was collected from all samples. These serum samples were investigated for the presence of neutralizing antibodies against FMD using a virus neutralization (VN) assay against the respective antigens. Group 1 received the benchmark vaccine stored at 4°C, Group 2 received vaccine A, stored at 4°C. Group 3 received vaccine A, stored at -18°C. Group 4 received vaccine B, stored at 4°C. Group 5 received vaccine B, stored at -18°C. Group 6 received vaccine C, stored at 4°C. Group 7 received vaccine C, stored at -18°C. The results are indicated below in Table 2.

**Table 2 Mean neutralizing antibody titers against FMD virus (titers in 10log)**

| | O1 Manisa | A Turkey | Asia 1 Shamir |
|---|---|---|---|
| Group 1 | 1.2 | 1.1 | 1.0 |
| Group 2 | 1.7 | 1.7 | 1.8 |
| Group 3 | 1.4 | 1.7 | 1.9 |
| Group 4 | 1.4 | 1.5 | 1.9 |
| Group 5 | 1.5 | 1.4 | 1.5 |
| Group 6 | 1.5 | 1.6 | - |
| Group 7 | 1.4 | 1.1 | - |

What can be seen is that there is no, or hardly any effect of the freezing on the potency of the water-in-oil emulsion adjuvanted vaccines, in complete contrast with the prior art that specifically teaches that FMD vaccines based on oily adjuvants cannot be frozen without detrimental effect on their potency. Since once frozen, the FMD antigens will hardly deteriorate or at least deteriorate at a speed significantly slower than in a vaccine stored at 4°C (all depending on the freezing and storage temperature, typically between -196°C and -12°C, e.g. at temperatures around -20 or -70°C which are typical for common freezers), an FMD vaccine based on a water-in-oil emulsion adjuvant may thus be sold with accompanying instructions (e.g. a label on the package, or a leaflet in the package) that the vaccine may be stored under frozen conditions.

## Claims

1. A method for keeping a foot-and-mouth disease vaccine available for emergency vaccination of animals against foot-and-mouth disease, the vaccine comprising a foot-and-mouth disease antigen formulated in a water-in-oil emulsion, said method comprising:
- providing the formulated vaccine,
- freezing the vaccine using a non snap-freezing process, and
- storing the frozen vaccine until it is needed for emergency vaccination.

2. A method according to claim 1, **characterised in that** a potency of the vaccine is established before it is stored.

3. A method according to claim 1 or 2, **characterised in that** the vaccine is supplied to a third party before it is frozen.

4. Method according to claim 1 or 2, **characterised in that** the vaccine is supplied to a third party after it has been frozen.

5. A method according to claim 1, **characterised in that** the vaccine is frozen in a compressor type freezer.

6. A method according to any of the preceding claims, **characterised in that** freezing takes place around -20°C.

7. A method according to any of the preceding claims, **characterised in that** the water-in-oil emulsion is based on mineral oil.

8. A method according to claim 7, **characterised in that** oil comprises saturated hydrocarbons.

## Patentansprüche

1. Verfahren zum Bereithalten eines Maul-und-Klauenseuche-Impfstoffs für Notfallimpfung von Tieren gegen Maul-und-Klauenseuche, wobei der Impfstoff ein in einer Wasser-in-Öl-Emulsion formuliertes Maul-und-Klauenseuche-Antigen umfasst, wobei das Verfahren umfasst:
- Bereitstellen des formulierten Impfstoffs,
- Einfrieren des Impfstoffs unter Verwendung eines Nicht-Schockgefrier-Verfahrens und
- Lagern des gefrorenen Impfstoffs, bis er für die Notfallimpfung benötigt wird.

2. Verfahren gemäß Anspruch 1, **dadurch gekennzeichnet, dass** eine Wirkungskraft des Impfstoffs nachgewiesen wird, bevor er gelagert wird.

3. Verfahren gemäß Anspruch 1 oder 2, **dadurch gekennzeichnet, dass** der Impfstoff einem Dritten geliefert wird, bevor er eingefroren wird.

4. Verfahren gemäß Anspruch 1 oder 2, **dadurch gekennzeichnet, dass** der Impfstoff einem Dritten geliefert wird, nachdem er eingefroren worden ist.

5. Verfahren gemäß Anspruch 1, **dadurch gekennzeichnet, dass** der Impfstoff in einer Gefriervorrichtung vom Kompressor-Typ eingefroren wird.

6. Verfahren gemäß einem der vorstehenden Ansprüche, **dadurch gekennzeichnet, dass** das Einfrieren bei etwa -20 °C erfolgt.

7. Verfahren gemäß einem der vorstehenden Ansprüche, **dadurch gekennzeichnet, dass** die Wasser-in-Öl-Emulsion auf Mineralöl basiert.

8. Verfahren gemäß Anspruch 7, **dadurch gekennzeichnet, dass** das Öl gesättigte Kohlenwasserstoffe umfasst.

## Revendications

1. Méthode destinée à maintenir la disponibilité d'un vaccin contre la fièvre aphteuse pour la vaccination d'urgence d'animaux contre la fièvre aphteuse, le vaccin comprenant un antigène de fièvre aphteuse formulé dans une émulsion eau-dans-huile, ladite méthode comprenant :
- la fourniture du vaccin formulé,
- la congélation du vaccin en utilisant un procédé différent d'une congélation rapide, et
- le stockage du vaccin congelé jusqu'à ce qu'on en ait besoin pour une vaccination d'urgence.

2. Méthode selon la revendication 1, **caractérisée en ce qu'**une antigénicité du vaccin est établie avant son stockage.

3. Méthode selon la revendication 1 ou 2, **caractérisée en ce que** le vaccin est fourni à un tiers avant sa congélation.

4. Méthode selon la revendication 1 ou 2, **caractérisée en ce que** le vaccin est fourni à un tiers après sa congélation.

5. Méthode selon la revendication 1, **caractérisée en ce que** le vaccin est congelé dans un congélateur à compression.

6. Méthode selon l'une quelconque des revendications précédentes, **caractérisée en ce que** la congélation a lieu à environ -20°C.

7. Méthode selon l'une quelconque des revendications précédentes, **caractérisée en ce que** l'émulsion eau-dans-huile est basée sur une huile minérale.

8. Méthode selon la revendication 7, **caractérisée en ce que** l'huile comprend des hydrocarbures saturés.
